# EUROPEAN PATENT SPECIFICATION

(11) **EP 3 285 874 B1**
(45) Date of publication and mention of the grant of the patent: **17.03.2021**
(21) Application number: 16784004.0
(22) Date of filing: 22.04.2016
(51) Int. Cl.: A61K 31/7036, A61K 31/7048, A61K 9/127, A61K 9/00, A61P 31/06, A61P 31/00, A61K 45/06

(54) **COMPOSITIONS AND METHODS FOR TREATING MYCOBACTERIA INFECTIONS AND LUNG DISEASE**
ZUSAMMENSETZUNGEN UND VERFAHREN ZUR BEHANDLUNG VON MYKOBAKTERIENINFEKTIONEN UND LUNGENERKRANKUNGEN
COMPOSITIONS ET MÉTHODES POUR LE TRAITEMENT D'INFECTIONS ET DE MALADIES PULMONAIRES À MYCOBACTÉRIES

(30) Priority: 22.04.2015 US 201562151218 P
(43) Date of publication of application: 28.02.2018
(73) Proprietor: Matinas BioPharma Nanotechnologies, Inc., Bedminster NJ 07921 (US)
(72) Inventor: LU, Ruying, New Providence, NJ 07974 (US)
(74) Representative: J A Kemp LLP
(86) International application number: PCT/US2016/028999
(87) International publication number: WO 2016/172595

(56) References cited:
- WO-A1-2012/151517
- WO-A1-2014/022414
- US-A1- 2009 169 635
- US-A1- 2014 220 108
- US-A1- 2014 315 842

## Description

### CROSS REFERENCE TO RELATED APPLICATIONS

This application claims the benefit of, and relies on the filing date of, U.S. provisional patent application number 62/151,218, filed 22 April 2015.

### FIELD

This application relates generally to encochleated aminoglycoside antibiotics to treat mycobacteria lung infections.

### BACKGROUND

Organisms of the *Mycobacterium* genus are widely distributed in the environment, and can be found forming biofilms in water pipes and potable water tanks. Some Mycobacteria species are highly virulent and may spread from host to host by coughing and inhalation or direct contact, causing leprosy (*M. leprae)* or tuberculosis (*M. tuberculosis*). Other Mycobacteria species are significantly less virulent and are collectively referred to as Non-Tuberculous Mycobacteria (NTM). Although NTM are less virulent, under certain circumstances they may infect hosts with weakened immune systems or a particular physiology (such as bronchiectasis). Such infection of the host may occur by two different routes. One is the gastro-intestinal route, from where the bacteria can disseminate or/and cause lymph node infection. The other is the respiratory route, by which the bacterium causes infection in individuals with chronic pulmonary conditions (bronchiectasis, emphysema, cystic fibrosis, chronic obstructive pulmonary disease). The latter route of infection is common in individuals with underlying lung disease, and the lung infection is associated with the formation of biofilm (Carter G, et al, AAC 48:4907, 2004; Yamazaki Y, et al Cell Microbiol,8: 808. 2006) WO 2012/151517 shows the *in vivo* efficacy of amikacin cochleates against disseminated infection caused by injection *of M. avium* complex. Moreover, this document describes a murine lung model of *M. avium* infection.

Amikacin (AMK) is an aminoglycoside antibiotic used most often to treat severe, hospital-acquired infections from multidrug-resistant gram-negative bacteria such as *Pseudomonas aeruginosa, Acinetobacter* and *Enterobacter,* as well as for the treatment of non-tuberculosis Mycobacterial (NTM) lung disease, typically for advanced lung disease and/or when first-line drugs fail to control the infection.

There is no oral form available as free amikacin is not absorbed orally, and amikacin, therefore must be given by intravenous (IV) or intramuscular (IM) routes. Liposomal amikacin for inhalation is currently in late stage clinical trials for the treatment of respiratory diseases, such as cystic fibrosis, *Pseudomonasaeruginosa,* non-tubercular mycobacterial infections and bronchiectasis.

Adverse side-effects of amikacin are similar to those of other aminoglycosides. Kidney damage and hearing loss are the most important effects. Because of this potential, blood levels of the drug and markers of kidney function (creatinine) are monitored.

Accordingly, the available routes for administering aminoglycoside antibiotics, like amikacin, are limited and their administration must be closely monitored due to toxicity concerns. Providing an oral formulation for aminoglycoside antibiotics like amikacin, and particularly one with a reduced toxicity profile, represents a major advancement in the treatment of mycobacterial infections, including NTM lung disease.

### SUMMARY

The invention is defined by the claims. Any subject-matter falling outside the scope of the claims is provided for information only. Any references in the description to methods of treatment refer to the compounds, pharmaceutical compositions or medicaments of the present invention for use in the treatment of the human or animal body.

The experiments disclosed in this application demonstrate that aminoglycoside (e.g., amikacin) cochleates are effective at killing *Mycobacteria avium* in biofilm and in an animal model of nontuberculosis mycobacterial (NTM) lung disease. Thus the disclosure is directed, in part, to methods of treating a subject with a *Mycobacterium avium* infection, the method comprising orally administering to the subject a therapeutically effective amount of a formulation comprising a cochleate, wherein the cochleate comprises an aminoglycoside antibiotic.

One embodiment is directed to a formulation comprising a cochleate, wherein the cochleate comprises an aminoglycoside antibiotic, for use in a method of treating a subject with a Mycobacteria lung disease, the method comprising orally administering to the subject a therapeutically effective amount of the formulation, and wherein bacterial load in the subject is reduced by at least 90% following treatment.

In one embodiment, the *Mycobacteria* lung disease is pulmonary tuberculosis. Typically, the pulmonary tuberculosis is caused by *M. tuberculosis, M. bovis, M. africanum, M. microti* and *M. canetti.*

In another embodiment, the *Mycobacteria* lung disease is nontuberculosis mycobacterial lung disease. In one embodiment, the NTM is selected from the group consisting of *Mycobacterium avium, M. kanasasii, M. abscessus, M.xenopi, M.* and *M. intracellulare,* and *Mycobacterium avium* complex.

In one embodiment, the method of treatment reduces bacterial load by at least 95%, or 98%. In one embodiment, the method of treatment reduces bacterial load by at least 90%, 95%, or 98% following at least 4 weeks of treatment.

In one embodiment, the subject has refractory lung disease following a standard course of therapy and before administration of the cochleate comprising the antibiotic.

In one embodiment, the subject has refractory lung disease following at least 6 months of a previous course of therapy and before administration of the cochleate comprising the antibiotic.

In one embodiment, the cochleate comprising the antibiotic is administered as monotherapy. In another embodiment, the cochleate comprising the antibiotic is administered as part of a multi-drug therapy.

In one embodiment, the multi-drug therapy comprises ethambutol and a macrolide, such as clarithromycin or azithromycin. In one embodiment, the multi-drug therapy further comprises rifamycin or rifampin.

In one embodiment, a no observed adverse effects level (NOAEL) of the aminoglycoside is greater than 100, 125, 150, 175, 200, 250, 300, 400, 500, 750, 1000, 1500, or 2000 mg/kg.

In one embodiment, the aminoglycoside antibiotic is selected from the group consisting of as amikacin, gentamycin, capreomycin, paromomycin, tobramycin, kanamycin, and neomycin. In one embodiment, the aminoglycoside antibiotic is amikacin.

In one embodiment, the antibiotic is administered at a dosage of between 5-20 mg/kg, alternatively 5-15 mg/kg, alternatively 5-10 mg/kg, alternatively 10-15 mg/kg, alternatively 10-20 mg/kg, alternatively 5-10 mg/kg, alternatively 5-25 mg/kg, or alternatively 1-30 mg/kg, In one embodiment, the antibiotic is administered at a dosage of about 400-1000 mg/day, alternatively 200-2000 mg/day, alternatively 100-4000 mg/day, alternatively 300-800 mg/day, alternatively 400-800 mg/day, alternatively 200-800 mg/day, alternatively 100-600 mg/day, alternatively 200-600 mg/day, alternatively 400-600 mg/day, alternatively 300-700 mg/day.

In one embodiment, the cochleate formulation further comprises sodium chloride. In one embodiment, the cochleate formulation contains 1 mM to 1M or 0.5M to 1M sodium chloride.

In one embodiment, the cochleate formulation further comprises bile salts. In one embodiment, the cochleate formulation contains 0.1mM to 100mM or 0.1mM to 0.5mM bile salts. In one embodiment, the bile salt is one or more of the following: cholate, chenodeoxycholate, taurocholate, glycocholate, taurochenodeoxycholate, glycochenodeoxycholate, deoxycholate, or lithocholate.

In one embodiment, the cochleate formulation is administered once per day, twice per day, three times per day, or four times per day. In another embodiment, the cochleate formulation is administered once per week, twice per week, three times per week, or four times per week. In one embodiment, the cochleate formulation is administered daily for at least 4 weeks. In another embodiment, the cochleate formulation is administered daily for at least 1 month, 2 months, 3 months, at least 4 months, or at least 6 months.

In one embodiment, the subject is a mammal. In one embodiment, the subject is a human.

In one embodiment, the cochleate comprises one or more negatively charged lipids, wherein the one or more negatively charged lipids comprise between 40% to 70% of the total lipid in the cochleate. In one embodiment, the one or more negatively charged lipids comprise between 30% to 70%, 40% to 60%, 45% to 65%, 45% to 60%, or 45% to 55% of the total lipid in the cochleate. In one embodiment, the one or more negatively charged lipids comprise phosphatidylserine. In one embodiment, the phosphatidylserine is soy phosphatidylserine. In another embodiment, the phosphatidylserine is egg or bovine derived phosphatidylserine.

In one embodiment, the cochleate further comprise one or more neutral or cationic lipid or sterols. In one embodiment, the one or more neutral or cationic lipid or sterols are selected from the group consisting of phosphatidylcholine and sphingomyelin.

In certain embodiments, the method is directed to a method of treating a subject with *Mycobacterium avium* complex (MAC) lung disease, the method comprising orally administering to the subject a therapeutically effective amount of a formulation comprising a cochleate, wherein the cochleate comprises an aminoglycoside antibiotic (e.g., amikacin).

Another aspect is directed to a method of treating a subject with nontuberculous mycobacterial (NTM) lung disease, the method comprising orally administering to the subject a therapeutically effective amount of a formulation comprising a cochleate, wherein the cochleate comprises an aminoglycoside antibiotic (e.g., amikacin).

Another aspect is directed to a method of treating a subject with pulmonary tuberculosis, the method comprising orally administering to the subject a therapeutically effective amount of a formulation comprising a cochleate, wherein the cochleate comprises an aminoglycoside antibiotic (e.g., amikacin).

Another aspect is directed to a method treating a subject with a *M*. *leprae* or *M. lepromatosis* lung infection, the method comprising orally administering to the subject a therapeutically effective amount of a formulation comprising a cochleate, wherein the cochleate comprises an aminoglycoside antibiotic (e.g., amikacin).

Yet another aspect is directed to a method of treating a subject with nontuberculous mycobacterial (NTM) lung disease, the method comprising orally administering to the subject a therapeutically effective amount of a formulation comprising a cochleate, wherein the cochleate comprises an aminoglycoside antibiotic (e.g., amikacin) and wherein the method of treatment reduces bacterial load by at least 90%, 95%, or 98%. In one embodiment, the method reduces bacterial load by at least 90%, 95%, or 98% following at least 4 weeks of treatment.

### BRIEF DESCRIPTION OF THE DRAWINGS

The accompanying drawings, which are incorporated in and constitute a part of this specification, illustrate certain embodiments, and together with the written description, serve to explain certain principles of the compositions and methods disclosed herein.
**Fig. 1** shows the effective treatment of *Mycobacterium avium* complex (MAC) biofilm in culture using amikacin cochleates.
**Fig. 2** shows the effective treatment of nontuberculosis Mycobacterium lung disease in mice treated daily for 4 weeks with amikacin cochleates.
**Fig. 3** shows that the no adverse effect limit (NOAEL) of encochleated amikacin in rats is twice as high as the NOAEL of injected amikacin.
**Figs. 4A-B** show the pharmacokinetics of encochleated amikacin in male (A) and female (B) rats. In humans, peak (Cmax) plasma levels should not exceed 35 mcg/mL and trough plasma levels should not exceed 10 mcg/mL, which levels are indicated by the upper horizontal lines in Figs. 4A-B.

### DETAILED DESCRIPTION

Reference will now be made in detail to various exemplary embodiments, examples of which are illustrated in the accompanying drawings and discussed in the detailed description that follows. It is to be understood that the following detailed description is provided to give the reader a fuller understanding of certain embodiments, features, and details of aspects of the invention.

### 1. Mycobacteria

Mycobacteria are a family of small, rod-shaped bacilli that can be classified into 3 main groups for the purpose of diagnosis and treatment. The first is *Mycobacterium tuberculosis* complex which can cause pulmonary tuberculosis and includes *M. tuberculosis, M. bovis, M. africanum, M. microti* and *M. canetti.* The second group is *M. leprae* and *M. lepromatosis,* which cause Hansen's disease or leprosy. The third group is nontuberculous mycobacteria (NTM), which include all the other mycobacteria that can cause lung disease resembling tuberculosis, lymphadenitis, skin disease, or disseminated disease. NTM include, but are not limited to, *M. avium* Complex (MAC), *M. avium, M. kansasii, M. abscessus, M. chelonae, M. fortuitum, M. genavense, M. gordonae, M. haemophilum, M. immunogenum, M. intracellulare, M. malmoense, M. marinum, M. mucogenicum, M. nonchromogenicum, M. scrofulaceum, M. simiae, M. smegmatis, M. szulgai, M. terrae, M. terrae* complex, *M. ulcerans,* and *M. xenopi.* MAC includes at least two mycobacterial species, *M. avium* and *M. intracellulare.* These two species cannot be differentiated on the basis of traditional physical or biochemical tests, but there are nucleic acid probes that can be used to identify and differentiate between the two species.

There are two major forms of NTM lung disease: cavitary disease and nodular/bronchiectatic disease. Cavitary disease typically presents in males in their late 40s and early 50s who have a history of cigarette smoking and, often, excessive alcohol consumption. If left untreated, this form of NTM lung disease is generally progressive within 1-2 years and can result in extensive cavitary lung destruction and respiratory failure. The nodular/bronchiectatic disease typically presents with nodular and interstitial nodular infiltrates frequently involving the right middle lobe or lingula, and typically in postmenopausal, nonsmoking, white females. This form of the disease tends to progress more slowly than the cavitary form.

In addition to NTM lung disease, NTM may also cause disseminated disease. Disseminated disease due to NTM is among the most common and severe infections found in individuals suffering from advanced HIV infection. The majority (greater than 90%) of NTM disseminated disease is caused by MAC, with the overwhelming majority of these cases caused by *M. avium.* Disseminated disease due to NTM in individuals infected with HIV typically only occurs in patients who are severely immunocompromised and have very low CD4+ T cell counts. Disseminated disease due to NTM has also been detected in immunocompromised organ transplant (e.g., renal or cardiac) patients or chronic corticosteroid use.

Pulmonary tuberculosis is an acute or chronic infection caused by *Mycobacteria tuberculosis* or sometimes other Mycobacteria strains (*M. bovis, M. africanum, M. microti* and *M. canetti*) and is characterized by pulmonary infiltrates, formation of granulomas with caseation, fibrosis, and cavitation.

Leprosy is a chronic, systemic infection characterized by progressive cutaneous lesions and caused by *M. leprae,* an acid-fast bacillus that attacks cutaneous tissue and peripheral nerves, producing skin lesions, infections, and deformities. Leprosy can also be caused by *M. lepromatosis.* Leprosy occurs in three distinct forms. Lepromatus leprosy is the most serious type and causes damage to the upper respiratory tract, eyes, and testes, as well as nerves and skin. Tuberculoid leprosy affects peripheral nerves and occasionally the surrounding skin, especially on the face, arm, legs, and buttocks. Borderline (dimorphous) leprosy has characteristics of both lepramatous and tuberculoid leprosies. Skin lesions in this type of leprosy are diffuse and poorly defined.

### 2. Cochleates and Methods of Making the Same

Cochleates are anhydrous, stable, multi-layered lipid crystals which spontaneously form upon the interaction of negatively charged lipids, such as phosphatidylserine, and calcium (see, for example, U.S. Pat. Nos. 4,078,052; 5,643,574; 5,840,707; 5,994,318; 6,153,217; 6,592,894, as well as PCT Publ. Nos. WO 200404/091572; WO 2004/091578; WO 2005/110361, WO 2012/151517, and WO2014/022414, and U.S. Pat. Publ. 2010/0178325). Cochleates have a unique multilayered structure consisting of a large, continuous, solid, lipid bilayer sheet rolled up in a spiral or as stacked sheets, with no internal aqueous space. This unique structure provides protection from degradation for associated "encochleated" molecules. Since the entire cochleate structure is a series of solid layers, components within the interior of the cochleate structure remain intact, even though the outer layers of the cochleate may be exposed to harsh environmental conditions or enzymes. Divalent cation concentrations *in vivo* in serum and mucosal secretions are such that the cochleate structure is maintained. Hence, the majority of cochleate-associated molecules are present in the inner layers of a solid, stable, impermeable structure. Once within the interior of a cell, however, the low calcium concentration results in the opening of the cochleate crystal and release of the molecule that had been formulated into cochleates. Accordingly, cochleate formulations remain intact in physiological fluids, including mucosal secretions, plasma and gastrointestinal fluid, thereby mediating the delivery of biologically active compounds by many routes of administration, including oral, mucosal and intravenous.

Cochleates can be made using known methods including, but not limited to, those described in U.S. Patent Nos. 5,994,318 and 6,153,217. In one embodiment, the method generally includes combining an aminoglycoside antibiotic with a lipid (preferably a negatively charged phospholipid, such as phosphatidylserine) in the presence of a solvent, adding an aqueous solution to form liposomes, and precipitating with a multivalent cation to form a cochleate. In another embodiment, the method generally includes combining an aminoglycoside antibiotic with a liposome in the presence of a solvent such that the aminoglycoside antibiotic associates with the liposome, and precipitating with a multivalent cation to form a pharmacologically active agent-containing cochleate.

In a preferred embodiment, the multivalent cation is a divalent metal cation, such as calcium, zinc, magnesium, and barium. In a preferred embodiment, the divalent metal cation is calcium.

The step of introducing an aminoglycoside antibiotic to a liposome in the presence of a solvent can be achieved in a variety of ways. In one embodiment, the aminoglycoside antibiotic is introduced by introducing a solution of the solvent and the aminoglycoside antibiotic to the liposome. Preferably, the liposome is in a liposomal suspension, preferably, an aqueous liposomal suspension. In a preferred embodiment, the solution with the antibiotic is introduced to the liposome by dropwise addition of the solution. In other embodiments, the solution can be added by continuous flow or as a bolus. In addition the solution may be introduced to dried lipid, with water added before, after or with the solution.

In another embodiment, the aminoglycoside antibiotic is introduced to the liposome prior to or after the solvent. For example, the aminoglycoside antibiotic may be introduced to a liposomal suspension that includes the solvent. The mixture can then be agitated, mixed, vortexed or the like to facilitate association of the aminoglycoside antibiotic with the liposome. The aminoglycoside antibiotic introduced may be in a powder or a liquid form.

An antioxidant (e.g., Vitamin E) can also be used in making cochleates. The antioxidant can be introduced with the aminoglycoside antibiotic or with the liposome. Preferably, it is incorporated into the liposomal suspension or a solution of the aminoglycoside antibiotic and solvent.

The liposome may be prepared by any known method of preparing liposomes. Thus, the liposomes may be prepared for example by solvent injection, lipid hydration, reverse evaporation, freeze drying by repeated freezing and thawing. The liposomes may be multilamellar (MLV) or unilamellar (ULV), including small unilamellar vesicles (SUV). The concentration of lipid in these liposomal solutions can be from about 0.1 mg/ml to 500 mg/ml. Preferably, the concentration of lipid is from about 0.5 mg/ml to about 50 mg/ml, more preferably from about 1 mg/ml to about 25 mg/ml.

The liposomes may be large unilamellar vesicles (LUV), stable plurilamellar vesicles (SPLV) or oligolamellar vesicles (OLV) prepared, e.g., by detergent removal using dialysis, column chromatography, bio beads SM-2, by reverse phase evaporation (REV), or by formation of intermediate size unilamellar vesicles by high pressure extrusion. Methods in Biochemical Analysis, 33:337 (1988).

Any suitable solvent can be used in these methods. Solvents suitable for a given application can be readily identified by a person of skill in the art. Preferably, the solvent is an FDA acceptable solvent. The solvent can be an organic solvent or an inorganic solvent. In one embodiment, the solvent is a water miscible solvent. In another embodiment, the solvent is water or an aqueous buffer. Other suitable solvents include but are not limited to dimethylsulfoxide (DMSO), a methylpyrrolidone, N-methylpyrrolidone (NMP), acetonitrile, alcohols, e.g., ethanol (EtOH), dimethylformamide (DMF), tetrahydrofuran (THF), and combinations thereof. In general, the aminoglycoside antibiotic concentration within the solvent is between about 0.01 mg/ml and 200 mg/ml. Preferably, the aminoglycoside antibiotic concentration is between about 0.05 mg/ml and about 100 mg/ml, more preferably between about 0.1 mg/ml and 20 mg/ml.

The solvent can optionally be removed, e.g., before the formation of liposomes, at the liposome stage and/or after the cochleates are formed. Any known solvent removal method can be employed. For example, solvent may be removed from the liposomal suspension by tangential flow and/or filtration and/or dialysis, or from the cochleates by washing, filtration, centrifugation, and/or dialysis. The cochleates can be washed, e.g., with buffer or water, optimally with calcium or another cation.

A size-regulating agent may be introduced during the method of making the cochleate. A size-regulating agent, as used herein, refers to an agent that reduces the particle size of a cochleate. As used herein, the term "particle size" refers to the particle diameter, or in case the particles are not spherical, to the largest extension in one direction of the particle. The particle size of cochleates can be measured using conventional methods, such as a submicron particle size analyzer. In certain embodiments, the size regulating agent is a lipid-anchored polynucleotide, a lipid-anchored sugar (glycolipid), or a lipid-anchored polypeptide. In other embodiments the size regulating agent is a bile salt, such as oxycholate, cholate, chenodeoxycholate, taurocholate, glycocholate, taurochenodeoxycholate, glycochenodeoxycholate, deoxycholate, or lithocholate. Bile salts are bile acids compounded with a cation, usually sodium. Bile acids are steroid acids found predominantly in the bile of mammals and are commercially available.

In certain embodiments, the size-regulating agent is added to the lipid or liposomes before formation of the precipitated cochleate. For example, in one embodiment, the size-regulating agent is introduced into a liposomal suspension from which cochleates will subsequently be formed (e.g., by addition of cation or dialysis). Alternatively, the size-regulating agent may be introduced to a lipid solution, before of after addition of an aminoglycoside antibiotic.

Any suitable lipid can be used to make the cochleate. In one embodiment, the lipid includes one or more negatively charged lipids. As used herein, the term "negatively charged lipid" includes lipids having a head group bearing a formal negative charge in aqueous solution at an acidic, basic or physiological pH, and also includes lipids having a zwitterionic head group.

The cochleates can also include non-negatively charged lipids (e.g., positive and/or neutral lipids). Preferably, the cochleates include a significant amount of negatively charged lipids. In certain embodiments, a majority of the lipid is negatively charged. In one embodiment, the lipid is a mixture of lipids, comprising at least 50% negatively charged lipid. In another embodiment, the lipid includes at least 75% negatively charged lipid. In other embodiments, the lipid includes at least 85%, 90%, 95% or 98% negatively charged lipid. In yet other embodiments, the negatively charged lipid comprises between 30%-70%, 35%-70%, 40%-70%, 45%-65%, 45%-70%, 40%-60%, 50%-60%, 45%-55%, 45%-65%, or 45%-50% of the total lipid in the cochleate.

The negatively charged lipid can include soy-based lipids, other-legume-based lipids, egg-based lipids, bovine-based lipids, or porcine-based lipids. Preferably, the lipid includes phospholipids, such as soy-based phospholipids. The negatively charged lipid can include phosphatidylserine (PS), dioleoylphosphatidylserine (DOPS), phosphatidic acid (PA), phosphatidylinositol (PI), and/or phosphatidyl glycerol (PG) and or a mixture of one or more of these lipids with other lipids. Additionally or alternatively, the lipid can include phosphatidylcholine (PC), phosphatidylethanolamine (PE), diphosphotidylglycerol (DPG), dioleoyl phosphatidic acid (DOPA), distearoyl phosphatidylserine (DSPS), dimyristoyl phosphatidylserine (DMPS), dipalmitoyl phosphatidylglycerol (DPPG) and the like. In one embodiment, the phosphatidylserine is soy phosphatidylserine. In another embodiment, the phosphatidylserine is egg or bovine derived phosphatidylserine.

### 3. Antibacterial Agents

The cochleates for use in the methods described herein are associated with or loaded with an antibacterial agent (also referred to herein as an antibiotic). The present invention relates to cochleates loaded with aminoglycoside antibiotics. In a further disclosure, by way of example, the anti-bacterial agent can include, but is not limited to, one or more of the following: a protein synthesis inhibitors; a 30S initiation inhibitor, such as aminoglycoside antibiotics (i.e. the antibiotics covered by present invention, including streptomycin, dihydrostreptomycin, neomycin, framycetin, paromomycin, ribostamycin, kanamycin, amikacin, arbekacin, bekanamycin, dibekacin, tobramycin, spectinomycin, hygromycin B, paromomycin, capreomycin, gentamicin, netilmicin, sisomicin, isepamicin, verdamicin, and astromicin); a 30S tRNA binding antibiotic, such as tetracyclines, glycylcyclines, or fluorocyclines (including doxycycline, chlortetracycline, clomocycline, demeclocycline, lymecycline, meclocycline, metacycline, minocycline, oxytetracycline, penimepicycline, rolitetracycline, tetracycline, tigecycline, or eravacycline); a 50S initiation inhibitor, such as oxazolidinone antibiotics (including eperezolid, linezolid, posizolid, radezolid, ranbezolid, sutezolid, and tedizolid); a peptidyl transferase, such as amphenicols or pleuromutilins (including chloramphenicol, azidamfenicol, thiamphenicol, florfenicol, retapamulin, tiamulin, and valnemulin); a transpeptidation/translocation antibiotic, such as macrolides, ketolides, fluoroketolides, lincosamides or streptogramins (including azithromycin, clarithromycin, dirithromycin, erythromycin, flurithromycin, josamycin, midecamycin, miocamycin, oleandomycin, okitamycin, roxithromycin, spiramycin, troleandomycin, tylosin, telithromycin, cethromycin, solithromycin, fidaxomicin, carbomycin A, kitasamycin, clindamycin, lincomycin, pirlimycin, pristinamycin, quinupristin, dalfopristin, and virginiamycin); an elongation factor inhibitor, such as steroid antibacterials (including fusidic acid); a peptidoglycan synthesis/transpeptidases inhibitor, such as a penicillin (including natural penicillins penicillin G and penicillin V; β-lactamase-resistant penicillins methicillin, nafcillin, oxacillin, cloxacillin and dicloxacillin; aminopenicillins ampicillin, amoxicillin, pivampicillin, hetacillin, bacampicillin, metampicillin, talampicillin and epicillin; carboxypenicillins carbenicillin, and ticarcillin; ureidopenicillins mezlocillin and piperacillin) or a cehphalosporin (including cephacetrile, cefadroxyl, cephalexin, cephaloglycin, cephalonium, cephaloradine, cephalothin, cephapirin, Cefatrizine, Cefazaflur, Cefazedone, cephazolin, cephradine, cefroxadine, ceftezole, cefaclor, cefonicid, cefprozil, cefuroxime, cefuzonam, cefmetazole, cefotetan, cefoxitin, loracarbef, cefbuperazone, cefmetazole, cefminox, cefotetan, cefoxitin, cefotiam, cefcapene, cefdaloxime, cefdinir, cefditoren, cefetamet, cefixime, cefmenoxime, cefodizime, cefotaxime, cefovecin, cefpimizole, cefpodoxime, cefteram, ceftamere, ceftibuten, ceftiofur, ceftiolene, ceftizoxime, ceftriaxone, cefoperazone, ceftazidime, latamoxef, cefclidine, cefepime, cefluprenam, cefoselis, cefozopran, cefpirome, cefquinome, flomoxef, ceftobiprole, ceftaroline, ceftolozane, cefaloram, cefaparole, cefcanel, cefedrolor, cefempidone, cefetrizole, cefivitril, cefmatilen, cefmepidium, cefoxazole, cefrotil, cefsumide, ceftioxide, cefuracetime, and nitrocefin); a penems or carbapenem (including faropenem, ertapenem, doripenem, imipenem, meropenem, biapenem, and panipenem); a monobactam (including aztreonam, tigemonam, carumonam, nocardicin A); a glycopeptide antibiotic (including vancomycin, oritavancin, telavancin, teicoplanin, dalbavancin, and ramoplanin); a beta-lactamase inhibitor (including clavulanate, sulbactam, tazobactam, and avibactam), an other antibiotic (including: fosfomycin, cycloserine, bacitracin, colistin, polymyxin B, daptomycin, lysozyme, gramicidin, isoniazid, or teixobactin).

In certain embodiments, the aminoglycoside antibiotic according to the invention is streptomycin, dihydrostreptomycin, neomycin, framycetin, paromomycin, ribostamycin, kanamycin, amikacin, arbekacin, bekanamycin, dibekacin, tobramycin, spectinomycin, hygromycin B, paromomycin, capreomycin, gentamicin, netilmicin, sisomicin, isepamicin, verdamicin, and astromicin. In one embodiment, the aminoglycoside is amikacin.

### 4. Pharmaceutical Compositions

The cochleates described herein can be prepared as a pharmaceutical composition. Suitable preparation forms for the pharmaceutical compositions disclosed herein include, for example, tablets, capsules, soft capsules, granules, powders, suspensions, emulsions, microemulsions, nanoemulsions, unit dosage forms, rings, films, suppositories, solutions, creams, syrups, transdermal patches, ointments and gels.

The pharmaceutical compositions can include other pharmaceutically acceptable excipients, such as, a buffer (e.g., Tris-HCl, acetate, phosphate) of various pH and ionic strength; an additive such as albumin or gelatin to prevent absorption to surfaces; a protease inhibitor; a permeation enhancer; a solubilizing agent (e.g., glycerol, polyethylene glycerol); an anti-oxidant (e.g., ascorbic acid, sodium metabisulfite, butylated hydroxyanisole); a stabilizer (e.g., hydroxypropyl cellulose, hydroxypropylmethyl cellulose); a viscosity increasing agent (e.g., carbomer, colloidal silicon dioxide, ethyl cellulose, guar gum); a sweetener (e.g. aspartame, citric acid); a preservative (e.g., Thimerosal, benzyl alcohol, parabens); ; a flow-aid (e.g., colloidal silicon dioxide), a plasticizer (e.g., diethyl phthalate, triethyl citrate); an emulsifier (e.g., carbomer, hydroxypropyl cellulose, sodium lauryl sulfate); a polymer coating (e.g., poloxamers or poloxamines, hypromellose acetate succinate); a coating and film forming agent (e.g., ethyl cellulose, acrylates, polymethacrylates, hypromellose acetate succinate); an adjuvant; a pharmaceutically acceptable carrier for liquid formulations, such as an aqueous (water, alcoholic/aqueous solution, emulsion or suspension, including saline and buffered media) or non-aqueous (e.g., propylene glycol, polyethylene glycol, and injectable organic esters such as ethyl oleate) solution, suspension, emulsion or oil; and a parenteral vehicle (for subcutaneous, intravenous, intraarterial, or intramuscular injection), including but not limited to, sodium chloride solution, Ringer's dextrose, dextrose and sodium chloride, lactated Ringer's and fixed oils.

In certain embodiments, the pharmaceutical composition comprises a salt, such as NaCl or a bile salt, such as oxycholate, cholate, chenodeoxycholate, taurocholate, glycocholate, taurochenodeoxycholate, glycochenodeoxycholate, deoxycholate, or lithocholate. Bile salts are bile acids compounded with a cation, usually sodium. Bile acids are steroid acids found predominantly in the bile of mammals and are commercially available. In one embodiment, the bile salts comprise cholate. In another embodiment, the bile salts comprises deoxycholate. In yet another embodiment, the bile salts comprise cholate and deoxycholate. In another embodiment, the bile salts consist of cholate and deoxycholate.

In certain embodiments, the concentration of NaCl is 1 mM to 1M, ImM to 0.5M, ImM to 0.1M, ImM to 50mM, 10mM to 100mM, 10mM to 50 mM, 0.1M to 1M, 0.1M to 0.5M, or 0.5M to 1M. In certain embodiments, the concentration of the bile salts is 1mM to 100mM, 1mM to 50 mM, 1mM to 25mM, 1 mM to 10mM, ImM to 5mM, 0.1mM to 5mM, 0.1mM to 1mM, or 0.1mM to 0.5mM bile salts.

These excipients are provided by way of example and it will be known to those of skill in the art that there will be other or different excipients that can provide the same chemical features as those listed herein.

### 5. Dosage and Administration

A pharmaceutical composition comprising a cochleate, as disclosed herein, is formulated to be compatible with its intended route of administration. The cochleate formulation according to the present invention is administered orally, for example, by administering a suspension, a tablet, a capsule, a softgel or other oral dosage form.

In certain embodiments, the antibiotic (e.g., an aminoglycoside, such as amikacin) is administered at a dosage of between 5-20, 5-10 mg/kg, 5-15, 10-15, 10-13 mg/kg, 10-20 mg/kg, 5-25 mg/kg, or 1-30 mg/kg. Alternatively, the antibiotic (e.g., an aminoglycoside, such as amikacin) can be administered at a fixed dosage of about 200-1000 mg/day, 400-1000 mg/day, 200-800 mg/day, 300-800 mg/day, 400-800 mg/day, 500-700 mg/day, 200-2000 mg/day, 100-4000 mg/day, 100-600 mg/day, 200-600 mg/day, 400-600 mg/day, or 300-700 mg/day,. including, but not limited to about 400, 500, 600, 700, 800, 900, 1000, 1500, 2000, 2500, 3000, 3500, or 4000 mg.

Due to lower toxicity, the encochleated antibiotic may be administered more frequently or for a longer duration than an intravenous antibiotic. In certain embodiments, the encochleated antibiotic may be administered once per day, twice per day, three times per day, or four times per day. In another embodiment, the cochleate formulation is administered once per week, twice per week, three times per week, or four times per week. In one embodiment, the encochleated antibiotic may be administered 2-3 times weekly. In other embodiments, the cochleate formulation is administered daily for at least 1, 2, 3, 4, 5, 6, 7, 8, 9, 10, 11, or 12 weeks. In another embodiment, the cochleate formulation is administered daily for at least 3 months, at least 4 months, or at least 6 months.

### 6. Methods of Treatment

In this section, any references to methods of treatment refer to the compounds, pharmaceutical compositions or medicaments of the present invention for use in the treatment of the human or animal body.

The cochleates as described herein can be used in a method of treating a subject with a Mycobacteria lung infection. One embodiment is directed to a method of treating a subject with a *Mycobacterium avium* lung infection, the method comprising orally administering to the subject a therapeutically effective amount of a formulation comprising a cochleate, wherein the cochleate comprises an aminoglycoside, including, for example, amikacin. In a disclosure, the methods can also be used to treat other mycobacterial infections.

One disclosure is directed to a method of treating a subject with disseminated nontuberculous mycobacterial (NTM) disease, the method comprising orally administering to the subject a therapeutically effective amount of a formulation comprising a cochleate, wherein the cochleate comprises an antibiotic, such as an aminoglycoside antibiotic (e.g., amikacin). Typically, the subject with disseminated NTM disease is an immunocompromised patient, such as a patient infected with HIV.

Another aspect is directed to a method treating a subject with a *M*. *leprae* or *M. lepromatosis* lung infection, the method comprising orally administering to the subject a therapeutically effective amount of a formulation comprising a cochleate, wherein the cochleate comprises an aminoglycoside antibiotic (e.g., amikacin).

Another aspect is directed to a method of treating a subject with *Mycobacteria* lung disease, the method comprising orally administering to the subject a therapeutically effective amount of a formulation comprising a cochleate, wherein the cochleate comprises an aminoglycoside antibiotic (e.g., amikacin). In one embodiment, the *Mycobacteria* lung disease is pulmonary tuberculosis. In another embodiment, the *Mycobacteria* lung disease is nontuberculosis mycobacterial (NTM) lung disease.

Thus, one embodiment is directed to a method of treating a subject with pulmonary tuberculosis, the method comprising orally administering to the subject a therapeutically effective amount of a formulation comprising a cochleate, wherein the cochleate comprises an aminoglycoside antibiotic (e.g., amikacin). Typically, pulmonary tuberculosis is caused by *M. tuberculosis, M. bovis, M. africanum, M. microti or M. canetti.*

Another embodiment is directed to a method of treating a subject with nontuberculosis mycobacterial (NTM) lung disease, the method comprising orally administering to the subject a therapeutically effective amount of a formulation comprising a cochleate, wherein the cochleate comprises an aminoglycoside, including, for example, amikacin. In certain embodiments, the NTM is selected from the group consisting of *Mycobacterium avium* complex, *M. kanasasii, M. abscessus, M.xenopi, M. avium,* and *M. intracellulare.* Typically, the NTM comprises at least *Mycobacterium avium* complex.

In certain embodiments, the method of treatment reduces bacterial load by at least 90%, 91%, 92%, 93%, 94%, 95%, 96%, 97%, 98%, 99%, or 100%. In certain embodiments, the method reduces bacterial load by at least 95%. In certain embodiments, the method reduces bacterial load by at least 98%. In one embodiment, the method reduces bacterial load by at least 90%, 91%, 92%, 93%, 94%, 95%, 96%, 97%, 98%, 99%, or 100% following at least 4 weeks of treatment. In certain embodiments, the method reduces bacterial load by at least 95% following at least 4 weeks of treatment. In certain embodiments, the method reduces bacterial load by at least 98% following at least 4 weeks of treatment.

Yet another aspect is directed to a method of treating a subject with nontuberculous mycobacterial (NTM) lung disease, the method comprising orally administering to the subject a therapeutically effective amount of a formulation comprising a cochleate, wherein the cochleate comprises an antibiotic, such as an aminoglycoside antibiotic (e.g., amikacin) and wherein the method of treatment reduces bacterial load by at least 90%, 91%, 92%, 93%, 94%, 95%, 96%, 97%, 98%, 99%, or 100%. In certain embodiments, the method reduces bacterial load by at least 95%. In certain embodiments, the method reduces bacterial load by at least 98%. In one embodiment, the method reduces bacterial load by at least 90%, 91%, 92%, 93%, 94%, 95%, 96%, 97%, 98%, 99%, or 100% following at least 4 weeks of treatment. In certain embodiments, the method reduces bacterial load by at least 95% following at least 4 weeks of treatment. In certain embodiments, the method reduces bacterial load by at least 98% following at least 4 weeks of treatment. In other disclosure the NTM disease is disseminated NTM disease.

In certain embodiments, the subject has refractory lung disease following a previous course of therapy and before administration of the cochleate comprising the antibiotic. As used herein, "refractory lung disease" refers to lung disease that does not respond (as measured microbiologically, clinically or radiographically) to an appropriate therapy. In certain embodiments, the subject has refractory lung disease following at least 1, 2, 3, 4, 5, 6, 7, 8, 9, 10, 11, or 12 months of a previous course of therapy and before administration of the cochleate comprising the antibiotic. Typically, the subject has refractory lung disease following at least 6 months of a previous course of therapy and before administration of the cochleate comprising the antibiotic. The previous course of therapy can be any therapy used for treating a Mycobacteria infection or NTM lung disease. Refractory lung disease can also refer to lung disease where sputum is converted to acid-fast bacilli culture negative after 12 months of an appropriate therapy.

Typically, the first-line or standard course of therapy for NTM lung disease comprises a multi-drug therapy with ethambutol and a macrolide, such as clarithromycin or azithromycin. The multi-drug therapy can further comprise rifamycin or rifampin. For more advanced disease or refractory disease, intravenous streptomycin or amikacin may be added to the multi-drug regimen.

Typically, the first-line or standard course of therapy for the nodular/bronchiectactic form of the disease typically includes a macrolide (e.g., clarithromycin, 1000 mg three times weekly or azithromycin, 500-600 mg three times weekly), ethambutol (25 mg/kg three times weekly), and rifampin (600 mg three times weekly).

Typically, the first-line or standard course of therapy for the cavitary form of the disease typically includes a macrolide (e.g., clarithromycin, 500-1000 mg daily or azithromycin, 250-300 mg daily), ethambutol (15 mg/kg daily), rifampin (450-600 mg daily), and optionally intravenous streptomycin or amikacin, typically for the first 2-3 months of therapy (e.g., 25 mg/kg 2-3 times weekly).

Typically, the first-line or standard course of therapy for advanced or severe or previously treated disease typically includes a macrolide (e.g., clarithromycin, 500-1000 mg daily or azithromycin, 250-300 mg daily), ethambutol (15 mg/kg daily), rifabutin (150-300 mg daily) or rifampin (450-600 mg daily), and intravenous streptomycin or amikacin, typically for the first 2-3 months of therapy (e.g., 25 mg/kg 2-3 times weekly).

Typically, the first-line or standard course of therapy for pulmonary tuberculosis includes one or more of isoniazid, rifampin, rifabutin, rifapentine, pyrazinamide, and ethambutol, which can be supplemented with intravenous or intramuscular streptomycin, amikacin, kanamycin, and capreomycin.

In certain embodiments, the cochleate comprising the aminoglycoside antibiotic (e.g., amikacin) is administered as monotherapy. In other embodiments, the cochleate comprising the aminoglycoside antibiotic (e.g., amikacin) is administered as part of a multi-drug therapy, including for example a standard course of therapy for treating NTM lung disease or pulmonary tuberculosis, such as the standard courses of therapy described herein.

In certain embodiments, the subject has relapsed following a previous course of therapy and before administration of the cochleate comprising the antibiotic. As used herein, "relapse" refers to recurrence of lung disease. In certain embodiments, the subject relapses following at least 1, 2, 3, 4, 5, 6, 7, 8, 9, 10, 11, or 12 months of a previous course of therapy and before administration of the cochleate comprising the antibiotic. The previous course of therapy can be any therapy used for treating a Mycobacteria infection, NTM lung disease, NTM disseminated disease, pulmonary tuberculosis, or leprosy, including the standard courses of therapy described above and those known in the art.

The subject is a human or a non-human mammal, such as a dog, a cat, or a farm animal. Typically, the subject is a human.

### 7. Reduced Toxicity

Oral administration of encochleated aminoglycosides, such as amikacin, exhibit reduced toxicity as compared to parenteral administration of non-encochleated aminoglycosides. Patients treated with parenteral, non-encochleated aminoglycosides must be under close clinical observation because of the potential ototoxicity and nephrotoxicity associated with their use. The safety of parenteral administration of non-encochleated aminoglycosides for treatment periods longer than 14 days has not been established.

For parenteral administration of non-encochleated aminoglycosides, neurotoxicity, manifested as vestibular and permanent bilateral auditory ototoxicity, can occur in patients with pre-existing renal damage and in patients with normal renal function treated at higher doses and/or for periods longer than those recommended. The risk of aminoglycoside-induced ototoxicity is greater in patients with renal damage. High frequency deafness usually occurs first and can be detected only by audiometric testing. Vertigo may occur and may be evidence of vestibular injury. Other manifestations of neurotoxicity may include numbness, skin tingling, muscle twitching and convulsions. The risk of hearing loss due to aminoglycosides increases with the degree of exposure to either high peak or high trough serum concentrations. Patients developing cochlear damage may not have symptoms during therapy to warn them of developing eighth-nerve toxicity, and total or partial irreversible bilateral deafness may occur after the drug has been discontinued. Aminoglycoside-induced ototoxicity is usually irreversible. Aminoglycosides are also potentially nephrotoxic. The risk of nephrotoxicity is greater in patients with impaired renal function and in those who receive high doses or prolonged therapy. The no observed adverse effects level (NOAEL) of intravenous amikacin is 100 mg/kg.

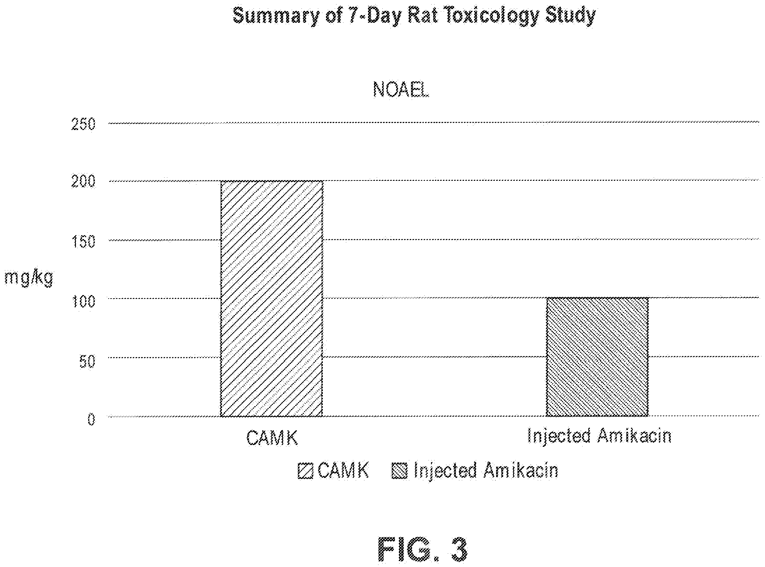

Administering aminoglycosides as part of a cochleate formulation reduces the toxicity associated with the aminoglycosides and permits the administration of higher doses of aminoglycosides. The NOAEL of a 7-day treatment regimen with encochleated amikacin in rats is 200 mg/kg or higher, two times the level of intravenous amikacin. Figure 3. In certain embodiments, the NOAEL of the administered encochleated antibiotic (e.g., an aminoglycoside, such as amikacin) is greater than 100, 125, 150, 175, 200, 250, 300, 400, or 500 mg/kg.

Due to toxicity concerns, in humans, the peak (Cmax) plasma levels of amikacin should not exceed 35 µg/mL and the trough plasma levels should not exceed 10 µg/mL. In certain embodiments, the Cmax of the administered encochleated antibiotic (e.g., an aminoglycoside, such as amikacin) is between about 100-500 ng/mL (0.1-0.5 µg/mL) or about 100-400 ng/mL (0.1-0.4 µg/mL). The lower toxicity and lower Cmax values for encochleated antibiotics, such as amikacin, permits the antibiotics to be delivered orally at lower doses with improved efficacy and reduced toxicity. Alternatively, due to the lower toxicity, the encochleated antibiotic can be administered more frequently and/or at higher doses with less risk of adverse consequences.

### EXAMPLES

The examples provided below are simply for illustrative purposes. Those of skill in the art will be able to readily determine appropriate methods and equipment in order to produce suitable solid dispersion forms as described herein.

### EXAMPLE 1: Cochleate Treatment of Mycobacteria Biofilm

**System:** A549 alveolar epithelial cells were cultured in a transwell system. A549 cells became polarized after 6 days and integrity. Bacteria were seeded on the top (apical surface) of the cells. Seven days were allowed for biofilm formation.

**Bacteria:** *Mycobacterium avium* complex (MAC) 104 (10⁵ bacteria) infection inoculum.

**Treatment:** 0.1 ml of the different treatments were delivered to the bottom well daily. The basolateral surface (bottom) of the cells were immersed in the tissue culture medium present in the bottom well. Three replicas per experimental group were tested in two different experiments.

**Harvesting:** Biofilm and epithelial cells were lysed and diluted and plated onto 7H10.

| **Experimental groups** | **CFU/bacteria, 14 days** | **P value** |
|---|---|---|
| No treatment | 7.4 ± 0.3 x 10⁶ | - |
| Empty cochleate | 6.9 ± 0.5 x 10⁶ | P > 0.05 |
| Free amikacin (100 µg/ml) | 3.2 ± 0.3 x 10⁵ | P = 0.02 |
| Free amikacin (20 µg/ml) | 9.1 ± 0.4 x 10⁵ | P < 0.05 |
| Cochleate-amikacin (0.66 M NaCl) 100 µg/ml | 5.7 ± 0.4 x 10⁵ | P = 0.02 |
| Cochleate-amikacin (0.66 M NaCl) 20 µg/ml | 1.8 ± 0.4 x 10⁶ | P < 0.05 |
| Cochleate-amikacin (2 mM bile salt) 100 µg/ml | 4.8 ± 0.3 x 10⁵ | P = 0.02 |
| Cochleate-amikacin (2 mM bile salt) 20 µg/ml | 8.0 ± 0.5 x 10⁵ | P < 0.05 |

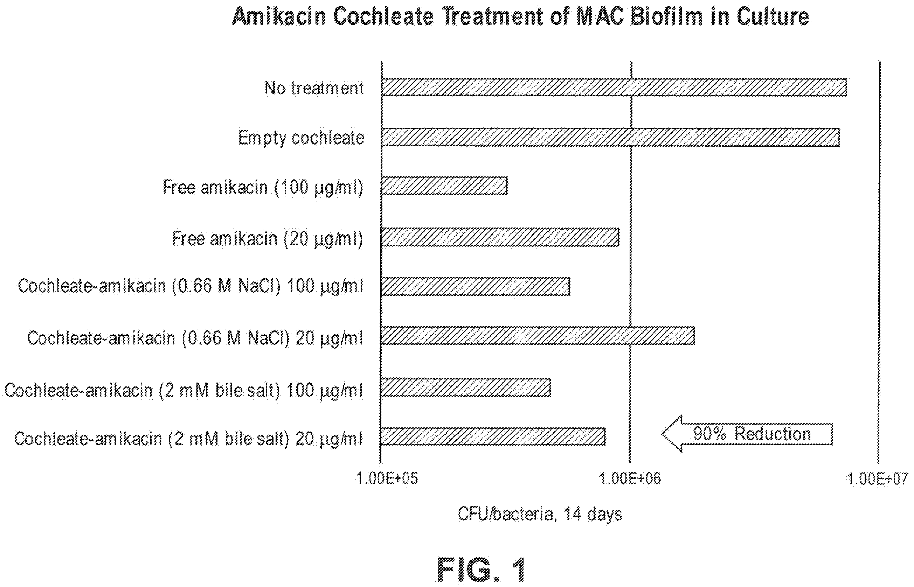

Conclusions: Biofilms of *M. avium* are encountered in lung infection. Although empty cochleate had no activity against *M. avium* in biofilm, both preparations of cochleates (sodium and bile salt) showed significant activity against *M. avium* in the model. Figure 1. The anti-bacterial activity of the cochleates was similar to the activity of free amikacin (Figure 1), suggesting that in absence of infected cells (small percent of the total infection), both preparations achieve comparable effect. Oral administration of encochleated amikacin shows anti-*M avium* activity in biofilm.

### EXAMPLE 2: Cochleate Treatment in Mice with Lung Disease

**Experimental model:** C57 BL/6 mice were infected with 8.3 x 10⁶ of *Mycobacterium avium* complex (MAC) intranasally and the infection was allowed to establish for 7 days. Then baseline bacterial load was determined in 10 mice and treatment protocols were initiated. Mice were treated daily for 4 weeks (orally or with intraperitonal injection of free amikacin). Mice were harvested and lung and spleens were removed, homogenized and plated to determine the bacterial load. Experimental groups had 12 mice each.

**Bacteria:** 8.3 x 10⁶ MAC 104/HBSS

**Results (bacterial load):**

| | |
|---|---|
| Baseline | 5.5 ± 0.4 x 10⁵ |
| Empty cochleate | 4.3 ± 0.6 x 10⁵ |
| Free amikacin oral | 2.2 ± 0.5 x 10^{5 (1)} |
| Free amikacin IP 100 mg/Kg | 5.6 ± 0.4 x 10^{3 (2) (3)} |
| Cochleate amikacin 100 mg/Kg/0.66 M NaCl | 9.8 ± 0.6 x 10^{3 (2) (3) (4)} |
| Cochleate amikacin 100 mg/Kg/2mM bile salt | 9.1 ± 0.4 x 10^{3 (2) (3) (4)} |
| Cochleate amikacin 100 mg/Kg/ washed +Ca⁺⁺ | 9.7 ± 0.3 x 10^{3 (2) (3) (4)} |

| | |
|---|---|
| (1) P> 0.05 compared to empty liposomes (2) P < 0.05 compared to empty liposomes (3) P < 0.05 compared to Baseline infection (4) Approximately 75% of amikacin incorporated into cochleates | |

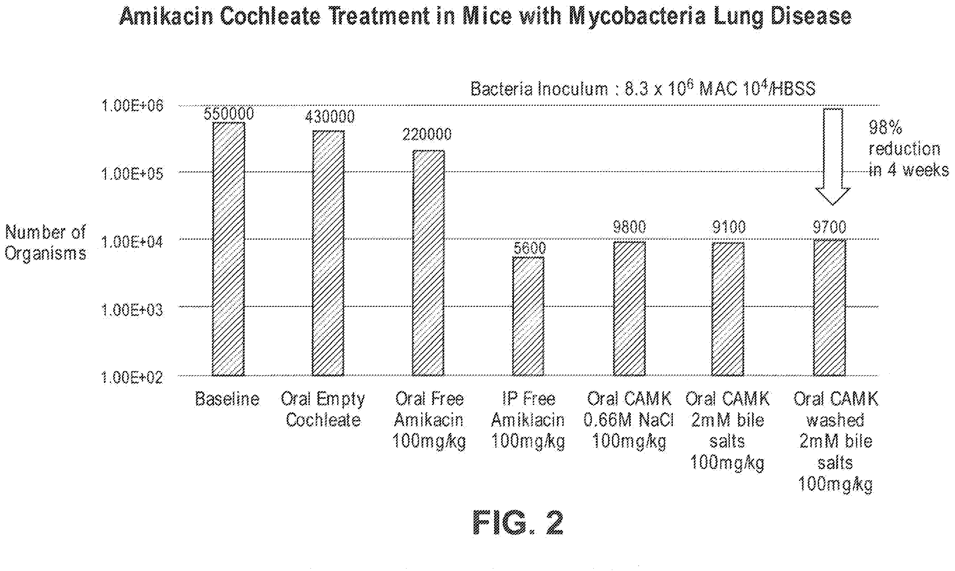

Free amikacin and cochleate amikacin preparations were effective for the treatment of lung infection by *M. avium* complex. Figure 2. The cochleate amikacin 100 mg/kg 0.66 M NaCl, cochleate amikacin 100 mg/kg 2 mM bile salt (unwashed) and cochleate amikacin 100 mg/kg (washed plus Ca++) had very similar reduced CFU loads at 4 weeks (9.1 to 9.8 x 10³), which were all significantly two orders of magnitude lower than empty cochleates and baseline infection. The free 100 mg/kg IP AMK group had a CFU load remaining that was approximately half (5.6 x 10³) of that observed in the CAMK treatment groups. The effect observed was bactericidal for all the orally administered cochleate preparations and the free amikacin administered IP. Surprisingly, oral CAMK reduced the bacterial load by 98% following 4 weeks of daily treatment (Figure 2), as compared to a reduction of only 76.3% (liver) and 86.7% (spleen) when oral CAMK was used to treat mice with disseminated *M. avium* infection. As noted above, the encochleation efficiency was about 75% for the CAMK treatment groups, which normalized the 100 mg/kg/day dose to 75 mg/kg/ day.

Histopathology results suggested a similar nontoxicity among the CAMK and free IP AMK preparations. Overall, no toxicity was observed (including the histopathology of kidneys).

In conclusion, the *in vivo* anti-infectivity experiments against MAC in the C57 BL/6 mouse lung infection model suggest that both of the 2 mM bile salt preparations and the high salt 0.66 NaCl formulation had comparable efficacy as the free 100 mg/kg IP AMK formulation. Histopathology results suggested a similar nontoxicity among CAMK prepared with bile salts or high salt 0.66 M NaCl. Overall, no toxicity was observed (including the histopathology of kidneys).

### EXAMPLE 3: In Vivo Toxicity Profile of Encochleated Amikacin

Amikacin (AMK) is a broad spectrum aminoglycoside commonly administered parenterally. Treatment with AMK is frequently associated with oto- and nephrotoxicity, therefore, careful monitoring of blood levels is required. To overcome issues associated with administration and toxicity of AMK, a cochleate formulation (CAMK) has been developed for oral delivery.

To assess the toxicity of CAMK, male and female Sprague Dawley rats (5/sex/group) were administered 50 mg/kg/day or 200 mg/kg/day oral dose of CAMK in 0.66 M NaCl, in 1mM bile salts, or in 2 mM bile salts for 7 days. Free AMK was administered at 200 mg/kg/day oral and at 50 mg/kg/day intravenous (iv). Rats were euthanized on Day 8. Parameters that were evaluated include mortality/morbidity, clinical observations, body weights, plasma drug levels, toxicokinetic analysis, clinical pathology, necropsy observations, organ weights, and histopathology.

All animals survived to their scheduled sacrifice. There were no significant test article-related clinical observations during the treatment period except fecal stain and soft stool, mainly in males. There were no significant body weight changes in CAMK-treated groups.

No changes in hematology parameters were observed in any of the orally-treated groups that were attributed to treatment with amikacin formulations. In addition, no microscopic changes in bone marrow or spleen were noted.

Treatment with generic amikacin at 50 mg/kg iv yielded a reduction in RBC and HGB with an associated decrease in MCHC as well as increases in REW, RET, and REA in males and decreases in MCH, RDW, RET and REA in females. These changes may indicate compensatory erythropoiesis, though again, direct effects on the bone marrow were not observed microscopically.

Modest increases in cholesterol, triglycerides, total protein and globulin were observed sporadically in some of the groups treated with amikacin. These changes were generally small and not clearly dose-related. Changes in some of these levels may be associated with changes in food consumption, but the lack of differences in body weights between groups argues against this hypothesis. These changes may be related to modest changes in the liver, and significant decreases in liver weights were observed in several groups; however, these changes were likewise not clearly dose-related, and decreased liver size is inconsistent with the increases seen in these parameters. There were no microscopic changes seen in the liver that suggested any adverse effects of any of the amikacin formulations on the liver, suggesting that all of these changes are of minimal toxicologic significance.

Organ weight changes were mainly observed in CAMK-NaCl groups and in generic amikacin-treated groups. Decreases of 10-16% in heart, kidney and liver weights with corresponding decreases in organ-to-body or organ-to-brain weight ratios (9.5-15%) were seen in females treated with CAMK-NaCl at 200 mg/kg. With generic amikacin (AMK), increases in kidney-and spleen-to-body weight ratios (10-22%) were observed in males in the 50 mg/kg generic amikacin iv group, but not in females. These findings were not considered to be of any toxicological significance because of the lack of histopathological changes within these organs.

CAMK in three different vehicle formulations and vehicle control (empty cochleates) was well-tolerated by Sprague Dawley rats at 50 and 200 mg/kg for 7 days with no apparent effects on mortality/morbidity, clinical observations, body weights, food consumption, ophthalmology, or clinical pathology (hematology, clinical chemistry, and coagulation). Across dose groups, oral CAMK provided 100 fold lower plasma levels of AMK than the iv dose. There was a trend toward a lower exposure on Day 7 versus Day 1 in several dose groups. The amikacin prescribing information states that the peak plasma levels (Cₘₐₓ) of amikacin should not exceed 35 µg/mL and the trough plasma levels should not exceed 10 µg/mL. The Cₘₐₓ of all CAMK formulations did not exceed 0.5 µg/mL (500 ng/mL). Figure 3.

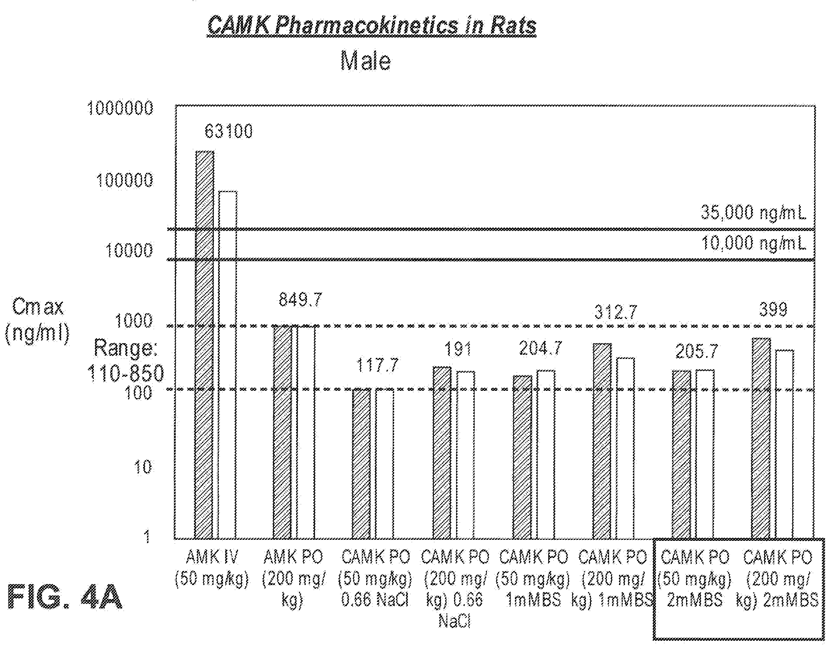

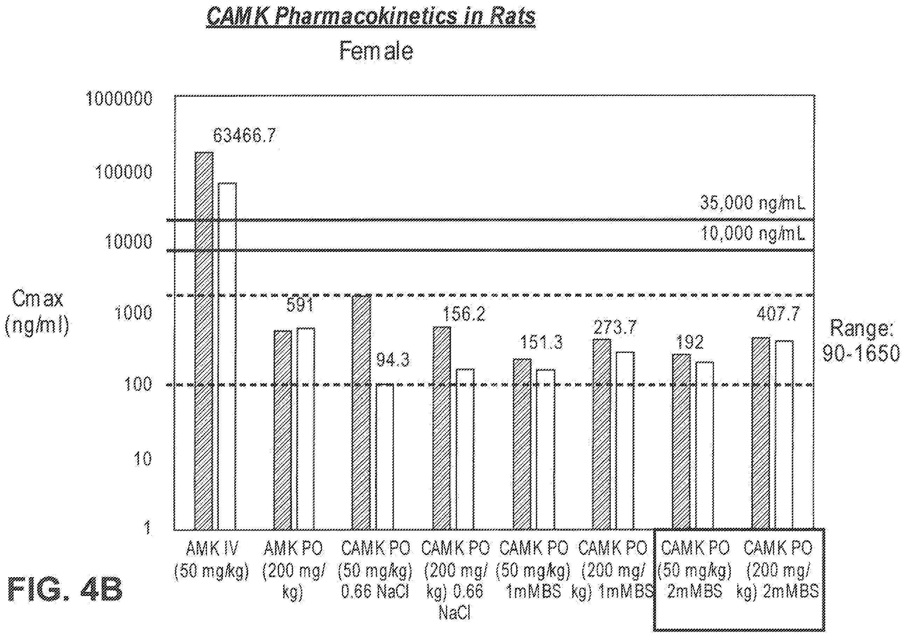

Based on changes in organ weights, the no adverse effect limit (NOAEL) is considered to be less than 50 mg/kg for CAMK in NaCl and 200 mg/kg for CAMK in 1 mM and 2 mM bile salt. Figure 4. This compares favorably with the published NOAEL for injected amikacin in rats, which is 100 mg/kg. Figure 4. The various changes seen in all treatment groups were of minimal toxicologic significance; therefore, the maximum tolerated dose (MTD) is considered to be greater than 200 mg/kg/day for all the dose formulations. This MTD is much higher than the currently recommended intravenous dose of 25 mg/kg three times weekly for patients with NTM lung disease.

Amikacin is typically administered intravenously (IV) or intramuscularly at 10-25 mg/kg/day for bacterial infections (approximately 300-500 mg as a single dose for a typical 70 kg human) to target plasma (or serum) peak and trough concentrations less than 35 µg/mL and 10 µg/mL, respectively. Following 7-day, repeat dose exposure of 200 mg/kg CAMK bile salts solution in rats, the systemic exposure of amikacin represented less than 2% of the systemic exposure following an IV 50 mg/kg dose. The NOAEL was considered to be 200 mg/kg for CAMK. Therefore, assuming similar oral bioavailability of CAMK in rats and humans, an 800 mg CAMK dose would be expected to produce peak concentrations in plasma less than the typical trough concentration observed following IV AMK, with trough CAMK concentrations at considerably lower levels. Using a conversion of 0.16 for body weight between rats and humans, the human equivalent dose (HED) of the NOAEL observed in rats would then translate to an HED of 32 mg/kg/day. For a typical 70 kg human, therefore, the NOAEL HED is 2240 mg/day. Applying a safety factor of approximately 10-fold, the minimum recommended starting dose is approximately 224 mg/day.

Those skilled in the art will recognize, or be able to ascertain using no more than routine experimentation, many equivalents to the specific embodiments of the invention described herein. Such equivalents are intended to be encompassed by the following claims.

## Claims

1. A formulation comprising a cochleate, wherein the cochleate comprises an aminoglycoside antibiotic, for use in a method of treating a subject with a *Mycobacteria* lung disease, the method comprising orally administering to the subject a therapeutically effective amount of the formulation, and wherein bacterial load in the subject is reduced by at least 90% following treatment.

2. The formulation for use of claim 1, wherein
(a) the *Mycobacteria* lung disease is pulmonary tuberculosis, preferably wherein the pulmonary tuberculosis is caused by *M. tuberculosis, M. bovis, M. africanum, M. microti* and *M. canetti;* or
(b) the *Mycobacteria* lung disease is nontuberculous mycobacterial (NTM) lung disease, preferably wherein the NTM is selected from the group consisting of *Mycobacterium avium, M. kanasasii, M. abscessus, M.xenopi, M. avium, M. intracellulare, and Mycobacterium avium* complex.

3. The formulation for use according to claim 1 or claim 2, wherein bacterial load in the subject is reduced by at least 95%, or 98% following treatment, and/or wherein bacterial load in the subject is reduced by at least 90%, 95%, or 98% following at least 4 weeks of treatment.

4. The formulation for use of any of the preceding claims, wherein the subject has refractory lung disease following a standard course of therapy and before administration of the cochleate comprising the aminoglycoside antibiotic, preferably wherein the subject has refractory lung disease following at least 6 months of a previous course of therapy and before administration of the cochleate comprising the aminoglycoside antibiotic.

5. The formulation for use of any of the preceding claims, wherein
(a) the cochleate comprising the aminoglycoside antibiotic is for administration as monotherapy, or
(b) the cochleate comprising the aminoglycoside antibiotic is for administration as part of a multi-drug therapy, preferably wherein the multi-drug therapy comprises ethambutol and a macrolide, such as clarithromycin or azithromycin, preferably wherein the multi-drug therapy further comprises rifamycin or rifampin.

6. The formulation for use of any of the preceding claims, wherein a no observed adverse effects level (NOAEL) of the aminoglycoside is greater than 100, 125, 150, 175, 200, 250, 300, 400, 500, 750, 1000, or 1500 mg/kg.

7. The formulation for use of any of the preceding claims, wherein the aminoglycoside antibiotic is selected from the group consisting of amikacin, gentamycin, paromomycin, capreomycin, tobramycin, kanamycin, and neomycin, more preferably wherein the aminoglycoside antibiotic is amikacin.

8. The formulation for use of any one of the preceding claims, wherein
(a) the aminoglycoside antibiotic is for administration at a dosage of between 5-20 mg/kg, 5-15 mg/kg, 5-10 mg/kg, 10-15 mg/kg, 10-20 mg/kg, 5-10 mg/kg, 5-25 mg/kg, or 1-30 mg/kg, and/or
(b) the aminoglycoside antibiotic is for administration at a dosage of about 400-1000 mg/day, 200-2000 mg/day, 100-4000 mg/day, 300-800 mg/day, 400-800 mg/day, 200-800 mg/day, 100-600 mg/day, 200-600 mg/day, 400-600 mg/day, or 300-700 mg/day.

9. The formulation for use of any one of the preceding claims, wherein the cochleate formulation further comprises sodium chloride, preferably wherein the cochleate formulation contains 1 mM to 1M or 0.5M to 1M sodium chloride.

10. The formulation for use of any one of the preceding claims, wherein the cochleate formulation further comprises bile salts, preferably wherein
(a) the cochleate formulation contains 0.1mM to 100mM or 0.1mM to 0.5mM bile salts; and/or
(b) the bile salt is one or more of the following: cholate, chenodeoxycholate, taurocholate, glycocholate, taurochenodeoxycholate, glycochenodeoxycholate, deoxycholate, or lithocholate.

11. The formulation for use of any one of the preceding claims, wherein
(a) the cochleate formulation is for administration once per day, twice per day, three times per day, or four times per day; and/or
(b) the cochleate formulation is for administration daily for at least at least 1 month, 2 months, 3 months, at least 4 months, or at least 6 months.

12. The formulation for use of any one of the preceding claims, wherein the subject is a mammal, preferably wherein the subject is a human.

13. The formulation for use of any one of the preceding claims, wherein the cochleate comprises one or more negatively charged lipids, wherein the one or more negatively charged lipids comprise between 40% to 70% of the total lipid in the cochleate, preferably wherein the one or more negatively charged lipids comprise between 45% to 60% of the total lipid in the cochleate, preferably wherein the one or more negatively charged lipids comprise phosphatidylserine, more preferably wherein the phosphatidylserine is soy phosphatidylserine.

14. The formulation for use of claim 13, wherein the cochleate further comprises one or more neutral or cationic lipid or sterols, preferably wherein the one or more neutral or cationic lipid or sterols are selected from the group consisting of phosphatidylcholine and sphingomyelin.

## Patentansprüche

1. Formulierung, die ein Cochleat umfasst, wobei das Cochleat ein Aminoglycosid-Antibiotikum zur Verwendung in einem Verfahren zur Behandlung eines Patienten mit einer *Mycobacteria-*Lungenerkrankung umfasst; wobei das Verfahren die orale Verabreichung einer therapeutisch wirksamen Menge der Formulierung an den Patienten umfasst und wobei die Bakterienbelastung in dem Patienten nach der Behandlung um mindestens 90% verringert ist.

2. Formulierung zur Verwendung nach Anspruch 1, wobei
(a) die *Mykobakterien-*Lungenerkrankung Lungentuberkulose ist, vorzugsweise wobei die Lungentuberkulose durch *M. tuberculosis, M. bovis, M. africanum, M. microti* und *M. canetti* verursacht wird; oder
(b) die *Mykobakterien*-Lungenerkrankung eine nicht tuberkulöse mykobakterielle (NTM) Lungenerkrankung ist, vorzugsweise wobei die NTM aus der Gruppe ausgewählt ist, die aus *Mycobacterium avium, M. kanasasii, M. abscessus, Mxenopi, M. avium, M. intracellulare* und dem *Mycobacterium avium*-Komplex besteht.

3. Formulierung zur Verwendung nach Anspruch 1 oder Anspruch 2, wobei die Bakterienbelastung in dem Patienten nach der Behandlung um mindestens 95% oder 98% verringert ist, und/oder wobei die Bakterienbelastung des Patienten nach mindestens 4-wöchiger Behandlung um mindestens 90%, 95% oder 98% verringert ist.

4. Formulierung zur Verwendung nach einem der vorhergehenden Ansprüche, wobei der Patient nach einem Standardtherapieverlauf und vor der Verabreichung des Cochleats, das das Aminoglycosid-Antibiotikum umfasst, eine refraktäre Lungenerkrankung aufweist, vorzugsweise wobei der Patient nach mindestens 6 Monaten eines vorherigen Therapieverlaufs und vor der Verabreichung des Cochleats, das das Aminoglycosid-Antibiotikum umfasst, eine refraktäre Lungenerkrankung aufweist.

5. Formulierung zur Verwendung nach einem der vorhergehenden Ansprüche, wobei
(a) das Cochleat, das das Aminoglycosid-Antibiotikum umfasst, zur Verabreichung als Monotherapie bestimmt ist oder
(b) das Cochleat, das das Aminoglycosid-Antibiotikum umfasst, zur Verabreichung im Rahmen einer Multidrogentherapie bestimmt ist, vorzugsweise, wobei die Multidrogentherapie Ethambutol und ein Makrolid wie Clarithromycin oder Azithromycin umfasst, vorzugsweise wobei die Multidrogentherapie ferner Rifamycin oder Rifampin umfasst.

6. Formulierung zur Verwendung nach einem der vorhergehenden Ansprüche, wobei ein Niveau von nicht beobachteten nachteiligen Wirkungen (No Observed Adverse Effects Level, NOAEL) des Aminoglycosids größer als 100, 125, 150, 175, 200, 250, 300, 400, 500, 750, 1000 oder 1500 mg/kg ist.

7. Formulierung zur Verwendung nach einem der vorhergehenden Ansprüche, wobei das Aminoglycosid-Antibiotikum aus der Gruppe ausgewählt ist, die aus Amikacin, Gentamycin, Paromomycin, Capreomycin, Tobramycin, Kanamycin und Neomycin besteht, wobei das Aminoglycosid-Antibiotikum bevorzugter Amikacin ist.

8. Formulierung zur Verwendung nach einem der vorhergehenden Ansprüche, wobei
(a) das Aminoglycosid-Antibiotikum zur Verabreichung in einer Dosierung zwischen 5-20 mg/kg, 5-15 mg/kg, 5-10 mg/kg, 10 - 15 mg/kg, 10-20 mg/kg, 5-10 mg/kg, 5 - 25 mg/kg oder 1-30 mg/kg bestimmt ist und/oder
(b) das Aminoglycosid-Antibiotikum zur Verabreichung in einer Dosierung von etwa 400 - 1000 mg/Tag, 200 - 2000 mg/Tag, 100 - 4000 mg/Tag, 300 - 800 mg/Tag, 400 - 800 mg/Tag, 200 - 800 mg/Tag, 100 - 600 mg/Tag, 200 - 600 mg/Tag, 400 - 600 mg/Tag oder 300 - 700 mg/Tag bestimmt ist.

9. Formulierung zur Verwendung eines der vorhergehenden Ansprüche, wobei die Cochleat-Formulierung ferner Natriumchlorid umfasst, vorzugsweise wobei die Cochleat-Formulierung 1 mM zu 1 M oder 0,5 M zu 1 M-Natriumchlorid enthält.

10. Formulierung zur Verwendung nach einem der vorhergehenden Ansprüche, wobei die Cochleat-Formulierung ferner Gallensalze umfasst, vorzugsweise wobei
(a) die Cochleat-Formulierung 0,1 mM bis 100 mM oder 0,1 mM bis 0,5 mM Gallensalze enthält; und/oder
(b) das Gallensalz eines oder mehrere der Folgenden ist: Cholat, Chenodesoxycholat, Taurocholat, Glycocholat, Taurochenodesoxycholat, Glycochenodesoxycholat, Desoxycholat oder Lithocholat.

11. Formulierung zur Verwendung nach einem der vorhergehenden Ansprüche, wobei
(a) die Cochleat-Formulierung einmal täglich, zweimal täglich, dreimal täglich oder viermal täglich verabreicht wird und/oder
(b) die Cochleat-Formulierung mindestens 1 Monat, 2 Monate, 3 Monate, mindestens 4 Monate oder mindestens 6 Monate lang täglich verabreicht werden muss.

12. Formulierung zur Verwendung nach einem der vorhergehenden Ansprüche, wobei der Patient ein Säuger ist, vorzugsweise wobei der Patient ein Mensch ist.

13. Formulierung zur Verwendung eines der vorhergehenden Ansprüche, wobei das Cochleat ein oder mehrere negativ geladene Lipide umfasst, wobei das eine oder die mehreren negativ geladenen Lipide zwischen 40% und 70% des gesamten Lipids in dem Cochleat ausmachen, vorzugsweise wobei das eine oder die mehreren negativ geladenen Lipide zwischen 45% und 60% des gesamten Lipids in dem Cochleat ausmachen, vorzugsweise wobei das eine oder die mehreren negativ geladenen Lipide Phosphatidylserin umfassen, bevorzugter, wobei das Phosphatidylserin Sojaphosphatidylserin ist.

14. Formulierung zur Verwendung nach Anspruch 13, wobei das Cochleat ferner ein oder mehrere neutrale oder kationische Lipide oder Sterole umfasst, vorzugsweise wobei das eine oder die mehreren neutralen oder kationischen Lipide oder Sterole aus der Gruppe ausgewählt sind, die aus Phosphatidylcholin und Sphingomyelin besteht.

## Revendications

1. Formulation comprenant un cochléate, dans laquelle le cochléate comprend un antibiotique aminoglycoside, pour une utilisation dans un procédé de traitement d'un sujet atteint d'une maladie pulmonaire à mycobactérie, le procédé comprenant l'administration orale au sujet d'une quantité thérapeutiquement efficace de la formulation, et dans laquelle une charge bactérienne chez le sujet est réduite d'au moins 90 % après traitement.

2. Formulation pour une utilisation selon la revendication 1, dans laquelle
(a) la maladie pulmonaire à mycobactérie est la tuberculose pulmonaire, de préférence dans laquelle la tuberculose pulmonaire est causée par *M. tuberculosis, M. bovis, M. africanum, M. microti* et *M. canetti* ; ou
(b) la maladie pulmonaire à mycobactérie est une maladie pulmonaire à mycobactérie non tuberculeuse (NTM), de préférence dans laquelle la NTM est choisie dans le groupe constitué par *Mvcobacterium avium, M. kanasasii, M. abscessus, M.xenopi, M. avium, M. intracellulare,* et un complexe de *Mycobacterium avium.*

3. Formulation pour une utilisation selon la revendication 1 ou la revendication 2, dans laquelle la charge bactérienne chez le sujet est réduite d'au moins 95 %, ou 98 % après traitement, et/ou dans laquelle la charge bactérienne chez le sujet est réduite d'au moins 90 %, 95 %, ou 98 % après au moins 4 semaines de traitement.

4. Formulation pour une utilisation selon l'une quelconque des revendications précédentes, dans laquelle le sujet est atteint d'une maladie pulmonaire réfractaire après un cycle thérapeutique classique et avant administration du cochléate comprenant l'antibiotique aminoglycoside, de préférence dans laquelle le sujet est atteint d'une maladie pulmonaire réfractaire après au moins 6 mois d'un cycle thérapeutique antérieur et avant administration du cochléate comprenant l'antibiotique aminoglycoside.

5. Formulation pour une utilisation selon l'une quelconque des revendications précédentes, dans laquelle
(a) le cochléate comprenant l'antibiotique aminoglycoside est destiné à une administration en monothérapie, ou
(b) le cochléate comprenant l'antibiotique aminoglycoside est destiné à une administration dans le cadre d'une thérapie multimédicamenteuse, de préférence dans laquelle la thérapie multimédicamenteuse comprend l'éthambutol et un macrolide, tel que la clarithromycine ou l'azithromycine, de préférence dans laquelle la thérapie multimédicamenteuse comprend en outre la rifamycine ou la rifampine.

6. Formulation pour une utilisation selon l'une quelconque des revendications précédentes, dans laquelle une dose sans effet nocif observé (NOAEL) de l'aminoglycoside est supérieure à 100, 125, 150, 175, 200, 250, 300, 400, 500, 750, 1000, ou 1500 mg/kg.

7. Formulation pour une utilisation selon l'une quelconque des revendications précédentes, dans laquelle l'antibiotique aminoglycoside est choisi dans le groupe constitué par l'amikacine, la gentamycine, la paromomycine, la capréomycine, la tobramycine, la kanamycine, et la néomycine, de manière davantage préférée dans laquelle l'antibiotique aminoglycoside est l'amikacine.

8. Formulation pour une utilisation selon l'une quelconque des revendications précédentes, dans laquelle
(a) l'antibiotique aminoglycoside est destiné à une administration à une posologie comprise entre 5 à 20 mg/kg, 5 à 15 mg/kg, 5 à 10 mg/kg, 10 à 15 mg/kg, 10 à 20 mg/kg, 5 à 10 mg/kg, 5 à 25 mg/kg, ou 1 à 30 mg/kg, et/ou
(b) l'antibiotique aminoglycoside est destiné à une administration à une posologie d'environ 400 à 1000 mg/jour, 200 à 2000 mg/jour, 100 à 4000 mg/jour, 300 à 800 mg/jour, 400 à 800 mg/jour, 200 à 800 mg/jour, 100 à 600 mg/jour, 200 à 600 mg/jour, 400 à 600 mg/jour, ou 300 à 700 mg/jour.

9. Formulation pour une utilisation selon l'une quelconque des revendications précédentes, dans laquelle la formulation de cochléate comprend en outre du chlorure de sodium, de préférence dans laquelle la formulation de cochléate contient du chlorure de sodium à 1 mM à 1M ou 0,5M à 1M.

10. Formulation pour une utilisation selon l'une quelconque des revendications précédentes, dans laquelle la formulation de cochléate comprend en outre des sels biliaires, de préférence dans laquelle
(a) la formulation de cochléate contient des sels biliaires à 0,1mM à 100mM ou 0,1mM à 0,5mM ; et/ou
(b) le sel biliaire est au moins l'un des suivants : cholate, chénodésoxycholate, taurocholate, glycocholate, taurochénodésoxycholate, glycochénodésoxycholate, désoxycholate, ou lithocholate.

11. Formulation pour une utilisation selon l'une quelconque des revendications précédentes, dans laquelle
(a) la formulation de cochléate est destinée à une administration une fois par jour, deux fois par jour, trois fois par jour, ou quatre fois par jour; et/ou
(b) la formulation de cochléate est destinée à une administration quotidienne pendant au moins 1 mois, 2 mois, 3 mois, au moins 4 mois, ou au moins 6 mois.

12. Formulation pour une utilisation selon l'une quelconque des revendications précédentes, dans laquelle le sujet est un mammifère, de préférence dans laquelle le sujet est un être humain.

13. Formulation pour une utilisation selon l'une quelconque des revendications précédentes, dans laquelle le cochléate comprend au moins un lipide à charge négative, dans laquelle l'au moins un lipide à charge négative comprend entre 40 % à 70 % du lipide total dans le cochléate, de préférence dans laquelle l'au moins un lipide à charge négative comprend entre 45 % à 60 % du lipide total dans le cochléate, de préférence dans laquelle l'au moins un lipide à charge négative comprend de la phosphatidylsérine, de manière davantage préférée dans laquelle la phosphatidylsérine est la phosphatidylsérine de soja.

14. Formulation pour une utilisation selon la revendication 13, dans laquelle le cochléate comprend en outre au moins un lipide ou stérol neutre ou cationique, de préférence dans laquelle l'au moins un lipide ou stérol neutre ou cationique est choisi dans le groupe constitué par la phosphatidylcholine et une sphingomyéline.
